(19) Europäisches Patentamt

European Patent Office

Office européen des brevets

(11) **EP 0 998 938 B1**

(12) **EUROPÄISCHE PATENTSCHRIFT**

(45) Veröffentlichungstag und Bekanntmachung des Hinweises auf die Patenterteilung:
**30.03.2005 Patentblatt 2005/13**

(51) Int Cl.$^7$: **A61K 35/66**, A61P 31/04

(21) Anmeldenummer: **99118983.8**

(22) Anmeldetag: **27.09.1999**

(54) **Antimikrobielle Mittel, Verfahren zu deren Herstellung und deren Verwendung**

Antimicrobials, methods of their manufacture and use thereof

Agents antimicrobiens, procédé pour leur obtention et leur utilisation

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **28.09.1998 DE 19844469**

(43) Veröffentlichungstag der Anmeldung:
**10.05.2000 Patentblatt 2000/19**

(73) Patentinhaber: **Herzog Mesmer, Artur, Dr. Dr.**
**60599 Frankfurt/Main (DE)**

(72) Erfinder:
• **Herzog Mesmer, Artur, Dr.Dr.**
**60599 Frankfurt/Main (DE)**
• **Iosiewitsch Lewi, Moisey, Prof.Dr.**
**60599 Frankfurt/Main (DE)**

• **Grigoriewitsch, Sutchkow Juri**
**129348 Moskau (RU)**

(74) Vertreter: **Kinzebach, Werner, Dr. et al**
**Patentanwälte**
**Reitstötter, Kinzebach und Partner**
**Postfach 86 06 49**
**81633 München (DE)**

(56) Entgegenhaltungen:
**EP-A- 0 333 177          EP-A- 0 405 569**
**WO-A-98/35014          DE-A- 2 819 035**

• **TEN BRINK, B., ET AL.: "Antimicrobial activity of lactobacilly: preliminary characterization and optimization of production of acidocin B, a novel bacteriocin produced by Lactobacillus acidophilus M46" JOURNAL OF APPLIED BACTERIOLOGY, Bd. 77, 1994, Seiten 140-148, XP002041005**

**Beschreibung**

Die vorliegende Erfindung betrifft neuartige, als Thermobiotika bezeichnete Mittel mikrobiellen Ursprungs mit antimikrobieller Aktivität, Verfahren zu deren Herstellung und deren Verwendung.

Bakterien und Pilze produzieren eine Fülle von Stoffen, die gemeinsam mit den sogenannten sekundären Pflanzenstoffen als Sekundärmetabolite bezeichnet werden. Viele dieser Substanzen besitzen beispielsweise Bedeutung als Therapeutika und Futtermittelzusätze. Als Produzenten von Sekundärmetaboliten haben insbesondere Mikroorganismen wissenschaftliche Bedeutung erlangt. Die bekanntesten Vertreter von Sekundärmetaboliten stellt die Gruppe der Antibiotika dar. Antibiotika sind Substanzen mikrobiellen Ursprungs, die schon in geringen Konzentrationen das Wachstum von Mikroorganismen hemmen. Man unterscheidet dabei zwischen lediglich hemmend wirkenden Substanzen (z.B. Bakteriostatika und Fungistatika) und abtötend wirkenden Substanzen (wie z.B. Bakterizide und Fungizide). Zur Bildung von Antibiotika sind hauptsächlich Pilze aus der Gruppe der Aspergillales sowie Bakterien aus der Gruppe der Actinomyceten befähigt.

Die Frage, welche Bedeutung die Antibiotaika für die sie produzierenden Organismen an ihrem jeweiligen Bodenstandort besitzen, kann derzeit nicht abschließend beantwortet werden. Charakteristisch für Sekundärmetabolite ist, dass die zu ihnen führenden Stoffwechselwege und Enzyme für Wachstum und Erhaltung der Zellen nicht benötigt werden. Ausgehend von der Annahme, dass während der Evolution immer nur das Sinnvolle Bestand hat, muss man in den Antibiotika Stoffe sehen, die den Produzenten an ihrem Standort einen Wachstumsvorteil, beispielsweise gegenüber Konkurrenten um das gleiche Substrat, einräumen.

Im Stand der Technik werden außerdem sogenannte Bakteriozine beschrieben. Hierbei handelt es sich um Proteine, welche von Bakterien gebildet werden und andere Bakterienstämme töten oder in ihrem Wachstum hemmen können. Diese Substanzen werden von Plasmiden kodiert. Bakteriozine sind beispielsweise aus E.coli (Colizine) isoliert worden.

Eine Besonderheit der Antibiotika besteht darin, dass sie die vegetativen Zellen anderer Mikroorganismen hemmen, jedoch nicht deren Sporen. Für die Bakteriozine ist charakteristisch, dass sie relativ spezifisch wirken und somit nur einen eng begrenzten Kreis verwandter Mikroorganismen inhibieren können.

Antimikrobiell wirkende Polypeptide werden zuweilen auch als Polypeptid-Antibiotika bezeichnet. Beispiele hierfür sind Subtilin sowie Nizin. Nizin ist aus 29 bis 34 Aminosäureresten aufgebaut und wird von Streptococcen produziert. Es besitzt bei erhöhter Temperatur (120°C) in saurem Medium (pH 2 bis 3) inhibierende Aktivität gegenüber Sporen von Bacillus stearothermophilus, wird jedoch selbst unter diesen Temperaturbedingungen sowie bei neutralem pH-Wert zersetzt. Nizin findet beispielsweise Anwendung als Konservierungsmittel zur Bekämpfung von Clostridien und Mycrobakterien bei der Käseherstellung. Subtilin besitzt ähnliche Eigenschaften wie Nizin und wird von Bacillus subtilis produziert. Praktische Anwendung hat Subtilin bisher nicht gefunden.

Insgesamt muss festgestellt werden, dass Anzahl und Eigenschaften der zur Verfügung stehenden antimikrobiell wirksamen Verbindungen bei weitem nocht nicht zufriedenstellend ist. Insbesondere besteht ein Bedarf an antimikrobiellen Mitteln, welche eine effektivere, d.h. raschere, Beseitigung bakterieller Sporen ermöglichen und gegen eine breites Spektrum von Mikroorganismen wirken.

Es ist deshalb Aufgabe der vorliegenden Erfindung neuartige antimikrobiell wirksame Verbindungen bereitzustellen, welche eine wirksame Bekämpfung unerwünschter Mikroorganisem auf eine breitere Basis stellen. Insbesondere sollten Mittel bereitgestellt werden, welche eine wirksamere und selektivere Bekämpfung von Mikroorganismen (das heißt von vegetativen Zellen und/oder Sporen dieser Mikroorganismen) ermöglichen.

Überraschenderweise wurde diese Aufgabe gelöst durch Bereitstellung neuartiger antimikrobiell wirkender Mittel, welche in bestimmter Weise nach Kultivierung von Bakterien erhältlich sind und sich dadurch auszeichnen, dass sie unter thermischen Bedingungen, insbesondere bei erhöhter Temperatur, antimikrobielle Aktivität besitzen, d.h. vegetative Zellen von Bakterien und/oder bakterielle Sporen und oder vegetative nichtbakterielle Zellen, wie z.B. Tumorzellen, in effizienterer Weise abtöten bzw. irreveribel inhibieren. Auf Grund ihrer charakteristischen antimikrobiellen Eigenschaften bei erhöhter Temperatur werden die erfindungsgemäßen Mittel im folgenden auch als "Thermobiotika" bezeichnet.

Erfindungsgemäß versteht man antimikrobielle Aktivität in einem erweiterten Sinn. Mikroben oder Mikroorganismen unfassen nämlich erfindungsgemäß Bakterien, Pilze, Viren, Sporen aber auch Zellen höherere eukaryotischer Systeme, wie z.B. gesunde oder krankhaft veränderte Zellen des menschlichen oder tierischen Körpers.

Ein erster Gegenstand der vorliegenden Erfindung betrifft ein Verfahren zur Herstellung eines erfindungsgemäßen antimikrobiell wirksamen Mittels, durch thermische Behandlung einer nach Kultivierung eines thermophilen, mesophilen oder psychrophilen, gram-positiven oder gram-negativen, aeroben oder anaeroben gegebenenfalls sporenbildenden Bakteriums und Waschen der Bakterienkultur erhältlichen Waschphase.

Eine erfindungsgemäß brauchbare, zur thermischen Behandlung geeignete Waschphase erhält man durch Waschen einer frisch hergestellten Bakterienkultur, wie z.B. einer bakteriellen Oberflächenkultur, mit einer wässrigen Waschflüssigkeit. Dazu trennt man die frisch gezüchteten Bakterien möglichst quantitativ vom Nähr- oder Kulturmedium ab und behandelt die Zellen in einem oder mehreren, vorzugsweise einem, Waschschritten mit der wässrigen, vorzugsweise gekühlten, Waschflüssigkeit. Nach jedem Waschschritt erfolgt die Abtrennung der behandelten Bakterienzellen durch Filtration oder vorzugsweise durch Zentrifugation.

Jedenfalls enthält die zur Herstellung eines antimikrobiellen Mittels geeignete Waschphase ein oder mehrere aus der Behandlung der Kultur mit der wässrigen Waschflüssigkeit und dem Separationsschritt resultierende Produkte, welche bei der folgenden thermischen Behandlung zu den neuartigen antimikrobiellen Mitteln führen.

Bevorzugt ist ein Verfahren zur Herstellung eines erfindungsgemäßen antimikrobiell wirksamen Mittels, durch

a) Kultivieren eines Bakteriums, vorzugsweise auf einem festen Nährmedium;

b) Abtrennen der Bakterienkultur vom Nährmedium und Waschen der Bakterienkultur mit einer wässrigen Waschflüssigkeit;

c) Abtrennen der dabei anfallenden flüssigen Waschphase von den Bakterien; und

d) thermische Behandlung der Waschflüssigkeit, vorzugsweise bei einer Temperatur von wenigstens etwa 120°C, vorzugsweise bei einem Druck von wenigstens etwa 1 Atmosphäre und vorzugsweise über einen Zeitraum von wenigstens etwa 5 min.

Die Durchführung des erfindungsgemäßen Herstellungsverfahrens ist nicht auf bestimmte Bakteriengattungen beschränkt. Erfindungsgemäß wurde nämlich überraschenderweise festgestellt, daß im wesentlichen alle Gattungen von Bakterien unabhängig von den jeweiligen morphologischen oder physiologischen Eigenschaften brauchbare Thermobiotika-Quellen darstellen. Auch der jeweilige Grad der Pathogenität des Bakteriums ist erfindungsgemäß ohne Belang. Zum Zwecke der weiteren Beschreibung der Erfindung werden die jeweils untersuchten Bakterien lediglich hinsichtlich ihrer Anforderung an die Wachstumstemperatur unterschieden. Erfindungsgemäß einsetzbare psychrophile Bakterien besitzen ein Wachstumsoptimum (das heißt eine maximale Wachstumsrate) unterhalb von etwa 20°C. Erfindungsgemäß brauchbare mesophile Bakterien besitzen ein Wachstumsoptimum bei einer Temperatur im Bereich von etwa 20 bis etwa 40°C. Erfindungsgemäß brauchbare thermophile Bakterien besitzen ein Wachstumsoptimum oberhalb von etwa 40°C, wie z.B. bei etwa 40 bis 110°C. Unter obige Definition brauchbarer Bakterien fallen auch solche Bakterien, welche als fakultativ thermophil, fakultativ mesophil oder fakultativ psychophil im Stand der Technik beschrieben werden. Die erfindungsgemäß einsetzbaren Bakterien sind außerdem unabhängig von ihrer Befähigung zur Sporenbildung verwendbar.

Nicht limitierende Beispiele für sporenbildende Bakterien sind ausgewählt unter B. stearothermophilus, B. subtilis, B. licheniformis und B. cereus. Nicht limitierende Beispiele für erfindungsgemäß brauchbare nicht-sporenbildende Bakterien sind ausgewählt unter E. coli, Y. pseudotuberculosis, Y. pestis, M. tuberkulosis, S. aureus und E. faecalis.

Für die Kultivierung verwendet man vorzugsweise ein festes Nährmedium, welchem die für das optimale Wachstum des jeweiligem Bakteriums erforderlichen Nährstoffe und Spurenelemente zugesetzt wurden. Erfindungsgemäß brauchbare feste Nährmedien sind Agar-Medien, welche Agar in einer Konzentration von etwa 15 bis 20 Gramm pro Liter enthalten. Die Herstellung geeigneter fester Nähremedien ist dem Fachmann geläufig. Die Kultivierung wird je nach Bakterium unter den jeweiligen optimalen Wachstumsbedingungen (Temperatur, Atmosphäre, Helligkeit, pH-Wert usw.) durchgeführt. Besonders bevorzugt ist die Herstellung von Oberflächenkulturen auf einem flächigen, festem Nährmedium, wie z.B. festen Agar-Platten.

Das erfindungsgemäße Herstellungsverfahren ist jedoch nicht auf eine Kultivierung auf festen Nährmedien beschränkt. Vielmehr eignet sich grundsätzlich auch die Kultivierung der Bakterien in halbfesten oder flüssigen Nährmedien. Auch diese Kulturtechniken und Methoden zur Isolierung der darin enthaltenen Bakterien sind dem Fachmann geläufig.

In einem nächsten Verfahrensschritt wird die erfindungsgemäße Bakterienkultur vom Nährmedium oder von der Kulturbrühe abgetrennt, und zwar vorzugsweise während der späten logarithmischen Wachstumsphase oder während der frühen stationären Wachstumsphase. Nach der Abtrennung wird mit einer wässrigen Waschflüssigkeit gewaschen. Als bevorzugte Waschflüssigkeit verwendet man erfindungsgemäß keimfreies, vorzugsweise destilliertes Wasser oder physiologische Kochsalzlösung. Die Entkeimung der Waschflüssigkeit kann beispielsweise durch Sterilisation oder Sterilfiltration erfolgen.

Dieser Waschschritt kann einmal oder mehrmals durchgeführt werden. Die Kultur wird dazu mit der Waschflüssigkeit in Kontakt gebracht. Die Waschflüssigkeit wird dann, gegebenenfalls nach kurzzeitiger Inkubation, vorzugsweise jedoch ohne weitere Inkubation von den Bakterien wieder getrennt. Dies erfolgt durch Filtration oder Zentrifugation. Zentrifugieren ist bevorzugt. Vorzugsweise zentrifigiert man bei einer relativen Zentrifugalbeschleunigung (RZB) von etwa 2000 g bis 30000 g, wie z.B etwa 2500 g bis 25000 g (berechnet nach der Formel: RZB = $1{,}119 * 10^{-5} * \text{Upm}^2 * r$ ; wobei Upm für die Zahl der Umdrehungen pro Minute und r für den Rotorradius steht). Vorzugsweise wird während der Zentrifugation z.B. auf 4 °C gekühlt. Die Zentrifugationsdauer beträgt z.B. 5 min bis 2 Stunden. Beispielsweise erfolgt die Zentrifugation 30 Minuten bei 2500g (entsprechend 3000 Upm bei einem Rotorradius von 25 cm) mit oder ohne Kühlung. Wichtig ist, daß die Bakterienzellen bei diesem Waschschritt nicht aufplatzen, so daß Zellinhalt in die Waschflüssigkeit austritt.

Der so erhaltenen zellfreie Überstand (d.h. die Waschphase) wird in einem weiteren Schritt einer thermischen Behandlung unterzogen. Bevorzugte Behandlungsbedingungen sind:

i) Temperatur: etwa 100 bis 200°C, wie z.B. 110 bis 180°C, insbesondere etwa 120°C;

ii) Druck: etwa 1 bis 3 Atmosphären, insbesondere etwa 1,5 bis 2 Atmosphären;

iii) Dauer: etwa 1 Minute bis 1 Stunde, insbesondere etwa 5 Minuten bis 30 Minuten, vorzugsweise etwa 5 bis 10 Minuten.

Die optimale Parameterkombination für obige Temperaturbehandlung kann mit Hilfe weniger Versuche festgestellt werden. Die Temperaturbehandlung kann beispielsweise durch Autoklavieren in herkömmlichen Autoklaviervorrichtungen erfolgen.

Das auf diese Weise durch thermische Behandlung der Waschphase erhältliche Produkt kann als solches zu den erfindungsgemäßen Zwecken eingesetzt werden.

Es besteht jedoch außerdem die Möglichkeit, die thermobiotische Aktivität in der Waschphase weiter einzugrenzen. Hierzu führt man beispielsweise eine Größenfraktionierung durch. Geeignete Größenfraktionierungsmethoden sind dem Fachmann bekannt. Beispielsweise besteht die Möglichkeit die Größenfraktionierung säulenchromatographisch durchzuführen. Geeignet ist jedoch auch die Größenfraktionierung durch größenselektive Filtration. So kann beispielsweise eine selektive Filtration mit Filter einer Porengröße von etwa 0,22 μm durchgeführt werden. Man erhält dabei als Filtrat ein aufbereitetes Thermobiotikum-Präparat deren Bestandteile eine Größe im Bereich von weniger als etwa 500 Dalton besitzen. Der oder die antimikrobiell aktiven Bestandteile dieses Präparates besitzen ein Molekulargewicht im Bereich von etwa 60 bis 450 Dalton, vorzugsweise etwa 60 bis 120 oder 80 bis 200 oder 100 bis 300 Dalton, wie z.B. 200 bis 300 Dalton. Vorzugsweise bestimmt man das Molekulargewicht dünnschichtchromatographisch (Stationäre Phase: Silikagel, Fa. Silufol, Tschechien; Mobile Phase: wässriger konzentrierter Ethylalkohol 95%ig; mit Harnstoff (etwa 60 Dalton) und Penicillin (etwa 300 Dalton) als Standards). Eine weitere Eingrenzung des antimikrobiell aktiven Bestandteils (der antimikrobiell aktiven Bestandteile) wäre zwar mit herkömmlichen präparativen Methoden möglich, ist jedoch aus praktischen Gründen nicht erforderlich.

Die erfindungsgemäß erhältliche thermisch behandelte, und gegebenenfalls größenfraktionierte Waschphase ist ohne Aktivitätsverlust bei etwa 2 bis 8 °C im flüssigen Zusand 6 Monate und länger und im getrockneten Zustand 36 Monate und länger lagerstabil.

Gemäß einer weiteren Variante des erfindungsgemäßen Herstellungsverfahrens wird die thermisch behandelte und gegebenenfalls größenfraktionierte Waschphase zur Trockne eingeengt. Als besonders geeignet hat sich hierzu die Trocknung der Flüssigkeitsphase bei 37°C, Normaldruck und über einen Zeitraum von mehreren Stunden bis mehreren Tagen erwiesen. Andere Trocknungsmethoden, wie z.B. die Gefriertrocknung wären ebenfalls brauchbar. Ein auf diese Weise hergestelltes Trockenpräparat kann vor der Anwendung in Wasser oder einem anderen geeigneten wässrigen Träger, wie z.B. einer physiologischen Kochsalzlösung oder einer Injektionslösung, rückstandsfrei wieder aufgelöst werden.

Ein weiterer Gegenstand der vorliegenden Erfindung betrifft die, beispielsweise nach einem der oben beschriebenen erfindungsgemäßen Verfahren erhältlichen, antimikrobiell wirksamen und als Thermobiotika bezeichneten Mittel. Diese sind insbesondere dadurch gekennzeichnet, dass deren aktiver Bestandteil (aktive Bestandteile) ein Molekulargewicht im Bereich von weniger als etwa 500 Dalton, wie z.B. etwa 60 bis 450 Dalton, wie z.B. 70 bis 400 Dalton, insbesondere etwa 100 bis 300 Dalton, besonders bevorzugt etwa 200 bis 300 Dalton besitzt (besitzen).

In funktioneller Hinsicht sind die antimikrobiell wirksamen Mittel vorliegender Erfindung dadurch gekennzeichnet, dass sie wenigstens eine antimikrobielle Aktivität besitzen, die ausgewählt ist unter:

a) einer inhibierenden Wirkung auf heterologe und/oder homologe Bakteriensporen; und

b) einer inhibierenden Wirkung auf heterologe und/oder homologe vegetative bakterielle und/oder vegetativ nichtbakterielle Zellen;

wobei die antimikrobielle Aktivität thermisch kontrollierbar ist, d.h. durch Temperaturerhöhung initiiert bzw. Temperaturabsenkung abgestellt werden kann.

Heterolog bedeutet in diesem Zusammenhang, daß das Thermobiotikum von einer anderen Bakterienspezies isoliert wurde als die vom Therobiotikum inhibierte Bakterien- oder Sporenspezies.

Vegetative Zellen sind Zellen ein- oder mehrzelliger pro- oder eukaryotischer Organismen, die zur Zellteilung durch Mitose befähigt sind.

Nichtbakterielle Zellen umfassen normale und insbesondere krankhaft veränderte Zellen, wie insbesondere Tumorzellen.

**EP 0 998 938 B1**

Eine erfindungsgmäße thermisch kontrollierbare Aktivität ist gegeben, wenn der antimikrobielle Effekt durch Temperaturerhöhung induzierbar ist. Insbesondere erfolgt die Temperaturerhöhung auf einen Wert, der über dem physiologischen Temperaturoptimum der zu inhibierenden Zielzellen oder Sporen liegt.

Insbesondere sind die erfindungsgemäßen Mittel vorzugsweise dadruch gekennzeichnet, dass sie vegetative Zellen (wie z.B. bakterille Zellen oder Körperzellen oder Tumorzellen) inhibieren, wenn man die Temperatur auf einen Wert in einem Bereich einstellt, dessen untere Grenztemperatur $T_u$ etwa der höchsten Temperatur entspricht, bie welcher die abzutötenden vegetativen Zellen noch vermehrungsfähig sind und dessen obere Grenztemperatur $T_o$ etwa der höchsten Temperatur entspricht, bei der diese Zellen noch lebensfähig aber nicht mehr vermehrungsfähig sind.

Erfindungsgemäß typische Temperaturbereiche für die Inhibierung vegetativer Zellen werden im folgenden angegeben, ohne jedoch darauf beschränkt zu sein:

- vegetative Zellen psychrophiler Bakterien: $T_u$ = 10°C $T_o$ = 45°C, wie z.B. 20 bis 40°C;
- vegetative Zellen mesophiler Bakterien: $T_u$ = 40°C $T_o$ = 70°C, wie z.B. 52 °C
- vegetative Zellen thermophiler Bakterien: $T_u$ = 70°C $T_o$ = 120°C, wie z.B. 75 bis 100 °C.
- Tumorzellen: $T_u$ = 41°C $T_o$ = 54°C, wie z.B. 43 bis 48°C, oder 44 bis 46°C oder 43,5 °C.

Erfindungsgemäße antimikrobiell wirksame Mittel mit Sporen-inhibierender Aktivität benötigen vorteilhafterweise zur Entfaltung ihrer Sporen-inhibierenden Wirkung eine Temperatur im Bereich von etwa 100 bis 150°C, vorzugsweise 110 bis 130°C, insbesondere etwa 120°C.

Weisen die erfindungsgemäßen Mittel eine Bakteriensporen-inhibierende Wirkung auf, so ist diese in charakteristischer Weise über einen weiten pH-Bereich, insbesondere über einen pH-Bereich von 2 bis 9, vorzugsweise etwa 6 bis 8, insbesondere 7 bis 7,5, im Unterschied zu dem aus dem Stand der Technik bekannten und oben beschriebenen Nizin nachweisbar.

Die inhibierende Wirkung auf vegetative bakterielle Zellen beobachtet man typischerweise in einem pH-Bereich von etwa 4 bis 9.

Die Dauer der Behandlung ist naturgemäß abhängig von der Temperaturwahl wie auch von der Konzentration des Thermobiotikums sowie von der Konzentration der zu inhibierenden Sporen oder vegetativen Zellen. Üblicherweise liegt die Dauer der Temperaturbehandlung jedoch in einem Bereich von 1 Minute bis 5 Stunden, vorzugsweise in einem Bereich von etwa 5 Minuten bis 1 Stunde, wie z.B. 5 bis 15 Minuten. Die Temperaturbehandlung von Tumorzellen (durch, vorzugsweise lokale, Hyperthermie) dauer üblicherweise 20 Minuten bis 4 Stunden, wie z.B. 2,5 Stunden, ein- oder mehrmals, wie z.B. 2- bis 4-mal pro Woche.

Ein erfindungsgemäßes antimikrobiell wirksames Thermobiotikum liegt in einer erfindungsgemäßen Präparation dann vor, wenn es als Aktivitätscharakteristikum zumindest Sporen von Bacillus stearothermophilus und/oder vegetative Zellen von Bacillus stearothermophilus und/oder vegetative Zellen von Escherichia coli inhibiert.

Die erfindungsgemäße Wirkung ist, wie durch die später beschriebene Ausführungsbeispiele noch genauer erläutert wird, nicht auf einen bestimmten Mikroorganismus bzw. Sporen eines bestimmten Mikroorganismus beschränkt. Vielmehr beobachtet man eine Vielzahl unterschiedlich ausgeprägter Aktivitätsmuster der erfindungsgemäßen Mittel. Allen Mitteln gemeinsam ist jedoch deren signifikante Aktivitätsentfaltung bei erhöhter Temperatur und deren thermische Stabilität.

Die Verwendung der erfindungsgemäßen Themobiotika auf den verschiedensten Gebieten der Medizin und der industriellen Technik sollte auf keine ernsten Hindernisse treffen. Die meisten der bisher untersuchten Thermobiotika wurden von nichtpathogenen Mikroorganismen erhalten, riefen bei Mäusen keine pathologischen Veränderungen hervor und zeigten eine gute Lagerstabilität. Selbst die aus Pathogenen erhaltenen Produkte ziegten keine pathogenen Effekte. Eine pharmakologische Anwendung der erfindungsgemößen Produkte ist daher angezeigt.

Gegenstand der vorliegenden Erfindung sind daher außerdem pharmazeutische Zusammensetzungen, welche in einem pharmazeutisch akzeptablen, festen oder flüssigen Träger eine wirksame Menge eines antimiktobiell wirksames Mittel gemäß obiger Definition enthalten.

Die Verabreichung der erfindungsgemäßen Mittel ist nicht auf bestimmte Applikationsformen beschränkt. Als Beispiele für geeignete Verabreichungswege sind zu nennen: intraarteriell, intravenös (z.B. durch Infusion), intramuskulär, intra- oder subcutan, oral, nasal, buccal, sublingual, retrobulbär, per inhalationem, vaginal, rektal, extracorporal, dermal, intradermal, peritoneal, intrathecal, intratumoral, elektrophoretisch, als Depotimplantat, über Katheter, sowie Verabreichung in Auge und Ohr.

Die für eine bestimmte pharmakologische Anwendung zu verabreichende Dosis ist von mehreren Faktoren abhängig, wie z.B. von der spezifischen antimikrobiellen Aktivität des jeweiligen Thermobiotika-Präparates, welche je nach Ursprungsorganismus unterschiedlich stark sein kann.

Weiterhin wird die Dosierung beeinflußt von der Art des zu bekämpfenden Bakteriums, von der Schwere der Erkrankung und vom Allgemeinzustand des zu behandelnden Patienten.

Weil Gegenindikationen für die erfindungsgemäßen Thermobiotika-Anwendungen sowie Nebenwirkungen der Präparate nicht bekannt sind, kann die zu verabreichende therapeutische Dosis auf der Grundlage des erzielten klinischen Effekts und der Fähigkeit der Patienten das Thermobiotikum aus dem Organismus auszuscheiden, bestimmt werden. Es gibt auch keine Gegenindikationen für die kombinierte Anwendung von Thermobiotika und Antibiotika sowie Thermobiotika und Chemotherapeutika, und insbesondere solcher Chemotherapeutika, die die Immunkräfte des Organismus schwächen und damit die Entstehung von Sekundärinfektionen fördern.

Im allgemeinen dürfte jedoch eine geeignete therapeutische Dosis im Bereich von etwa 5 bis 800 µg Thermobiotikum-Präparat je Kilogramm Körpergewicht liegen.

Die erfindungsgemäßen Thermobiotika können einzeln oder in Kombination mit anderen pharmakologisch wirksamen substanzen verwendet werden. Nichtlimitierende Beispiele für besonders geeignete Wirkstoffe sind: Nifedipin, Plasmin, Plasminogen, Betablocker, Acetylsalicylsäure, Prednisolon, Phenobarbital, Paracetamol-Panadol, EDTA, Heparin, Chinin, Atropin, Theophylin, Corticosteroide jeder Art, Antikonvulsiva jeder Art, Digitalis, Verapamil, Antidepressiva jeder Art, Antibiotika jeder Art, Antiepileptika, zentrale Analgetika, Parasympatholytika, Antiparkinsonmittel, Antihistaminika, trizyklische Antidepressiva, Diuretika, Antidiabetika, Muskelrelaxantien aller Art, Aminoglutethimid, Dexamethason, Digitoxin, Asparaginase, Medroxyprogesteronacetat, Megestrolacetat, ACE-Hemmer, Azathioprin, Allopurinol, Etoposid, Cyclophosphamid, Cyclosporin A, Lipopolysaccharide jeder Art, Doxorubicin, Daunorubicin, Diclofenac, Dipetiazem, Famotidin, Fluconazol, Halothan, Clipizid, Griseofulvin, Indometacin, Inthraconazol, Josamycin, Ketoconazol, Levonantrodol, Mesna, Melphalan, Antifolate, Methotrexat, Morphine und Opiate jeder Art, Omeprazol, Rifampicin, Sulfadiazin, Pentostatin, Pethidin, Thymidin, Procarbazin, Ranitidin, Hydroxyharnstoff, Tamoxifen, Idarubicin, Amphotericin B, Fotemustin, Chlorambucil, Immunstoffe aller Art, Interleukine aller Art, Immunotoxine, Ricin-Pertusis, Diphterietoxin, Interferonalpha, -beta und -gamma, Östrogene, Phenazon, Warfarin, Vinonelbin, Navelbin, Selen und selenhaltige Verbindungen, Streptozotocin, Thiole, G-SH-Acetyl-G-SH, Trimethoprim, Sulfadiacin, Vinblastin, Vincristin, Cimetidin, Epirubizin, Idarubizin, Mitoxantron, Estramustinphosphat, Thiotepa, Procarbazin, Nitrosoharnstoffe, CCNU, ME-CCNU, BCNU, ACNU, PCZ, UM-26, VP-16, Hexitolderivate, DAG, DBD, DIAC, Butyl-Scopolamin, Baclofen, Opioide, Dipidolor, Tramadol, Chlormezanon, Codein, Clonazepam, Dehydrocodein, Clonidin, Morphin, Hydromorphin, L-Polamidon, L-Methadon, Fentanyl, Damidronat, Haloperidol, Buskopan, Metamizol, Cytarabin, Dapson, Bleomycin, Acetyldigoxin, Cisplatin, Carboplatin, Platinpräparate und Platinsalze jeder Art, Phenytoin, Sauerstoff, Lachgas, Busulfan, Aminoglukoside, Ifosfamid, Natriumthiosulfat, Carmustin, Polio- und BCSCH-Impfstoffe und Impfstoffe aller Art, Chlorozotocin, Mitomycin C, Superoxidbismutase, Adreamycin, Plasmaproteine, Salicylate, Levamisol, Pyridoxin, Tauromustin, Fluorouracil, Phtoraphur, Calciumfolinat, Cystein, Dacarbacin, Monoaminoxidasehemmstoffe, Hexamethylmelamin, Lomustin, Semustin, Mercaptopurin, Cotrimoxazol, Nukleoside und Nukleosidanaloga jeder Art, Alkohol, Aminophenazon, Antirheumatika, Glafenin, Ketoprofen, Phenylbutazon, Prednison, Probenecid, Mitomycin, Diphosphonate, Calcitonin, Glucagon, Furosemid, Glycylpressin, Mitoxantron, N- Acetylcystein, Ceftacidim, Paclitaxel, Taxol, Chinidin, Ketonazol, Vitamine A, B, C, D und E, Antioxidantien jeder Art, Blutfarbstoffe jeder Art, Procarbazin, Sargromostim, Lithium, Phenytoin, Toremifen, Jomot, Jomotech, Jomoin, Purinantagonisten, Fluorarabinphosphate, Mexiticin, Midazolam, Zuckerlösung, Tetrazepam, Propanadol, Pindolol, Klophelin, Kordaron, Sotalol, Magnesium, Spironolacton, Triamteren, Aminoglycoside, DNA- und RNA-Extrakte aus Stöhr und Kalbsthymus, Betulinsäure, Akridonund Akridonverbindungen, Gluthathion (GSH) und GSH-Derivate.

Geeignete Darreichungsformen erfindungsgemäßer Thermobiotika umfassen z.B. wässrige Lösungen, wässrige Infusionen, Liposomenpräparate, Salben, Cremes, Präparate mit verzögerter Wirkstoffabgabe, wie Implantate oder Wirkstoffpflaster und dergleichen.

Gegenstand der Erfindung sind außerdem Desinfektionslösungen, enthaltend in einem üblichen zur Desinfektion geeigneten, flüssigen, inerten Träger ein antimikrobiell wirksame Menge eines wenigstens Mittels gemäß obiger Definition. Gewünschtenfalls können die erfindungsgemäßen Mittel in Kombination mit anderen aus dem Stand der Technik bereits bekannten und zum Zweck der Desinfektion bereits verwendeten Substanzen eingesetzt werden. Außerdem ist es denkbar, Kombinationen erfindungsgemäßer Mittel unterschiedlicher Herkunft (psychrophil/mesophil, psychrophil/thermophil, oder mesophil/thermophil) einzusetzen.

Die für eine Desinfektion einzusetzende Dosis ist von mehreren Faktoren abhängig, wie z.B. von der spezifischen antimikrobiellen Aktivität des jeweiligen Thermobiotika-Präparates, welche je nach Ursprungsorganismus unterschiedlich stark sein kann. Weiterhin wird die Dosierung beeinflußt von der Art des zu bekämpfenden Bakteriums und von der Schwere der Kontamination. Im allgemeinen dürfte jedoch eine geeignete desinfizierende Dosis im Bereich von etwa 10 bis 100 mg Thermobiotikum-Präparat je Liter Desinfektionslösung liegen.

Zum Zwecke der Konservierung von Nahrungsmittel sollten die erfindungsgemäßen Präparate in etwa in einer Dosis von 1 bis 8 mg/l Nahrungmittel verwendet werden.

Ein weiterer Gegenstand der Erfindung betrifft Wirkstoffkombinationen, umfassend wenigstens ein erfindungsgemäßes Thermobiotikum und wenigstens eine oberflächenaktive, vorzugsweise kartionische Substanz; sowie deren Verwendung zu den hierin beschriebenen Zwecken, insbesondere als antibakterielle Mittel. Es wurde nämlich erfindungsgemäß überraschenderweise beobachtet, daß derartige Kombinationen von Substanzen einen signifikant verbesserten antimikrobiellen Effekt aufweisen. Grundsätzlich sind alle üblichen oberflächenaktiven Substanzen brauchbar, solange sie nicht die Wirksamkeit des Thermobiotikums negativ beeinflussen. Nichtlimitierende Beispiele für geeignete oberflächenaktive Substanzen sind Ammoniumsalze der allgemeinen Formel $R^1N(R^2)_3{}^+X^-$, worin $R^1$ für einen langkettigen, geraden oder verzweigten Kohlenwasserstoffrest mit bis zu 25 Kohlenstoffatomen steht, die Reste $R^2$ gleich oder verschieden sind und für ein Wasserstoffatom oder einen Kohlenwasserstoffrest mit bis zu 4 Kohlenstoffatomen, welcher gegebenenfalls mit 1, 2, 3 oder 4 Hydroxylgruppen substituiert sein kann, stehen, oder zwei der Reste $R^2$ zusammen mit den Stickstoffatom an das sie gebunden sind für einen 4- bis 7- gliedrigen, gegebenenfalls aromatischen, heterozyklischen Ring stehen, und X für ein übliches Gegenion, insbesondere ein Halogenidion, wie z. B. Cl oder Br, steht. Beispiele sind insbesondere Mono-$C_8$-$C_{20}$- Akylammoniumsalze oder $C_8$-$C_{20}$-Alkyl-Pyridinium-salze, wie z.B. Cetylpyridiniumchlorid. Das oberflächenaktive Mittel kann in der Thermobiotika-Präparation in einem Anteil von 0,001 bis 0,1, vorzugsweise etwa 0,005 bis 0,05 Gew.-% enthalten sein. Das Thermobiotikum-Präparat kann unverdünnt oder in einer Verdünnung von 1:10 bis 1:1000 in üblichen Verdünnungsmitteln, wie z.B. Wasser, mit dem oberflächenaktiven Mittel vermischt werden.

So konnte beispielsweise festgestellt werden, daß ein erfindungsgemäßes Thermobiotikum aus Bacillus thermophilus oder aus einem 1:1 Gemisch (Zellen) von Bacillus stearothermophilus und E. coli nach 100-facher Verdünnung und Zugabe von 0,01 Gew.-% Cetylpyridiniumchlorid $10^6$ B. stearothermophilus-Sporen bzw. E. coli-Sporen in einem Zeitraum von 20 bis 30 min bereits bei 75°C abtötet. Unter den gleichen Versuchsbedingungen (Temperatur, Zeit, Konzentration) konnte für die Einzelkomponenten (Thermobiotikum bzw. oberflächenaktive Substanz) kein vergleichbarer antibakterieller Effekt nachgewiesen werden.

Gegenstand der Erfindung ist auch die Verwendung eines erfindungsgemäßen Thermobiotikums zur Herstellung einer pharmazeutischen Zusammensetzung zur Behandlung oder Prävention lokaler oder systemischer bakterieller Infektionen bei Mensch oder Tier. Als Beispiele können genannt werden eine Verwendung zur Behandlung von Verbrennungen, Rotlauf, Hautentzündungen, urogenitalen Infekten, chronischen Infekten innerer Organe, Infektionen des Nasen-Rachenraums, der Augenhöhlen oder des Innenohrs, und zur Behandlung von Sekundärinfektionen; besonders bevorzugt in Kombination mit einer begleitenten lokalen oder systemischen Hyperthermie-Behandlung.

Gegenstand der Erfindung ist auch die Verwendung erfindungsgemäßer Thermobiotika oder Mittel zur Desinfektion oder Sterilisation von Nahrungsmitteln oder medizinischen Geräten oder zur Konservierung von Lebensmitteln.

Besonders zweckmäßig ist die Verwendung erfindungsgemäßer Thermobiotika bei der Herstellung von den empfindlichen Nahrungsmitteln, die eine Wärmesterilisierung erfordern, und eine Verkürzung der Expositionsdauer die Qualität des Nahrungsmittels weiter erhöht.

Eine bevorzugte Ausführungsform betrifft die Verwendung erfindungsgemäßer Mittel zu pharmakologischen Zwecken in Kombination mit einer weiteren Therapieform, ausgewählt unter Chemotherapie, Strahlentherapie und Hyperthermie.

In Kombination mit einer lokalen oder allgemeinen Hypertherie können die Thermobiotika für die Behandlung von akuten und chronischen Ansteckungskrankheiten verwendet werden, wie z.B. Prostatitis, Pielonephritis, Pneumococceninfektionen oder Infektionen, wie Pneumonie.

Ein breites Feld für die Anwendung erfindungsgemäßer Thermobiotika umfaßt die Behandlung primärer und sekundärer Immunodefizit-Zustände, z.B. bei AIDS und ähnlichen Krankheitszuständen, sowie bei der Tumortherapie mit Zytostatika, wo häufig bedrohliche bakterielle Begleitinfektionen auftreten.

Außerdem wurde überraschenderweise festgestellt, daß die erfindungsgemäßen antimikrobiellen Mittel auch eine inhibierende Aktivität gegenüber Neoplasmen besitzen. Dieser inhibierende Effekt manifestiert sich in einer Suppression des Tumorwachstums und/oder einer Teil- oder Vollregression bereits ausgebildeter Tumoren. Insbesondere beobachtet man diesen Effekt bei Verabreichung erfindungsgemäßer Thermobiotika unter hyperthermischen Bedingungen. Zur weiteren Steigerung der Antitumoraktivität kann eine Verabreichung des Thermobiotikums zusammen mit herkömmlichen Antitumorwirkstoffen erfolgen, welche die Widerstandskraft der Tumorzellen senken. Als Beispiele für zur Kombination geeignete Wirkstoffe können genannt werden 5-Fluoruracil, Phtoraphur, Cyclophosphan, Endoxan, Methylhydrazin, Natulan, Procarbazin und Dacarbazin.

Gegenstand der Erfindung ist daher außerdem eine Verwendung erfindungsgemäßer Mittel zur Herstellung einer pharmazeutischen Zusammensetzung zur Behandlung von soliden oder nichtsoliden, hoch oder niedrig differenzierten Tumoren sowie Blutoder Lympherkrankungen verschiedener Genese, insbesondere in Kombination mit Hyperthermie.

Strahlentherapeutische Maßnahmen, wie die Bestrahlung mit Gammastrahlen, Laserstrahlen oder ultraviolettem Licht lassen sich mit einer erfindungsgemäßen Thermobiotika-Therapie gut kombinieren, weil diese physikalischen Faktoren die Beständigkeit der Mikroorganismen gegen die Thermobiotika herabsetzen.

Weil die Effektivität der Thermobiotika-Therapie durch Erhöhung der Körpertemperatur gesteigert werden kann, ist auch die gleichzeitige oder zeitlich vorgezogene Verabreichung fieberfördernder Mittel, wie z.B. von Pyrogenal, Polysacchariden und anderen Stoffen, zweckmäßig. Eine begleitende hyperthermische Behandlung ist besonders bevorzugt.

Weil die erfindungsgemäßen Thermobiotika vom Organismus ziemlich schnell (ca. 2 bis 10 h) ausgeschieden werden, ist die Anwendung deponierender Thermobiotika-Präparate mit zeitlich verzögerter Wirkstoffabgabe insbesondere bei chronischen Leiden angezeigt.

Um die Effektivität einer Thermobiotika-Therapie zu optimieren, sollte vorzugsweise vor einer Behandlung zuerst das Ansprechen der pathogenen Mikroflora auf die Thermobiotika bestimmt werden.Dies erleichtert die Wahl des optimalen Thermobiotikum-Präparats. Für sporenbildende Bakterien sollten z.B. solche Thermobiotika verwendet werden, die nicht nur die vegetativen Zellen, sondern auch deren Sporen inhibieren.

Unter Berücksichtigung der Fähigkeit der erfindungsgemäßen Thermobiotika bei erhöhter Temperatur Tumoren zu beseitigen, ist es erforderlich, deren Aktivität gegen verschiedene Neoplasmen zu prüfen, inbesondere in Kombination mit anderen physikalischen und chemischen Hilfsmitteln, welche die Widerstandskraft der Tumorzellen schwächen können.

Für die Thermobiotika-Anwendung ist die (lokale oder systemische) Erhöhung der Körpertemperatur Wirksamkeitsvoraussetzung. Das Niveau der Temperaturerhöhung und die Expositionsdauer hängen von den Eigenschaften des Applikationspunkts der Thermobiotika ab, sowie davon, wie der Patient die erhöhte Temperatur verträgt. Methoden und Vorrichtungen zur hyperthermischen Behandlung des menschlichen oder tierischen Körpers sind dem Fachmann bekannt.

Die Erfindung wird nun anhand der folgenden nichtlimitierenden Beispiele weiter erläutert.

Verwendete Testmethoden:

Beim Studium der Thermobiotika-Aktivität wurden hauptsächlich drei Methoden verwendet.

Methode 1: Bestimmung der antimikrobiellen Aktivität in Abhängigkeit von der Dauer der Sterilisation

In Glas- oder Plastikbehältern wurden Sporen und Thermobiotika in gleichen Volumina (0,01+0,01 ml) vorgelegt. In jedem Testansatz befand sich eine identische Sporenmenge (z.B. $10^6$). Die Behälter trocknete man 24 Stunden bei 37°C. Die Biotests wurden vor dem Autoklavieren in spezielle Beutel eingelegt. Die Autoklavierdauer betrug meistens 0, 5, 10, 15, 10, 25 und 30 Minuten bei gleicher Temperatur (beginnend nach Luftabsaugung aus der Sterilisationskammer). Für jede Autoklavierdauer verwendete man mindestens 5 biologische Tests. Folgende Kontrolltests wurden ebenfalls überprüft: 1) Biotests, die im Autoklaven nicht behandelt wurden; 2) Biotests aus einem Sporen-Wasser-Gemisch (als "Wasser" diente das Spülwasser vom unbesäten Agarmedien); 3) Behälter mit Nährmedium (1,5 μl). Nach dem Autoklavieren wurde jedem Biotest zum Nachweis lebensfähiger Sporen 0,5 ml eines flüssigen indikatorhaltigen Nährmediums folgender Zusammensetzung zugefügt:

| | |
|---|---|
| Trockenes Fleischpepton-Nährboullion | 15 g |
| Glucose | 5 g |
| Bromkresolgrün | 2 ml |
| (1%-ige alkoholische Lösung) | |
| H$_2$O dest. | ad 1 Liter |
| pH 7,6 ± 0,2, autoklaviert 20 min. bei 121 °C | |

Die Biotests wurden dann je nach getesteter Sporenspezies bei optimaler Temperatur inkubiert. Zum Nachweis von Sporen der Thermophils B. stearothermophilus erfolgte beispielsweise eine 48-stündige Inkubation bei 55°C. Lagen lebensfähige Sporen in einem Biotest vor, so beobachtete man einen Farbumschlag nach gelb.

Die rechnerische Auswertung der erhaltenen Ergebnisse erfolgte durch Bestimmung des Aktivitätsindex (A) aus der Gesamtzahl positiver Resultate (Farbumschlag nach gelb) bei den Kontrollansätzen (P) und bei den Thermobiotika-haltigen Testansätzen (Q) anhand der Formel:

$$((P-Q)/P) *100 = A$$

Es wurden immer gleichviele Kontroll- und Testansätze überprüft.

Analog verfuhr man bei Versuchen des gleichen Typs, die jedoch mit vegetativen Zellen anstatt mit Sporen durchgeführt wurden.

Methode 2: Bestimmung der antimikrobiellen Aktivität in Abhängigkeit von der Mikrobenkonzentration

Hierbei wurden verschiedene Mengen von Mikroben (Vegetative Zellen oder Sporen) mit Thermobiotika vermischt. Nach Einengen zur Trockne wurden die Biotests einer erhöhten Temperatur ausgesetzt. Anschließend wurden die Ansätze mit obigem Nährmedium versetzt und bei geeigneter Temperatur (z.B. 55 °C) kultiviert. Ein Farbumschlag des Indikators nach gelb zeigte die Anwesenheit von Sporen an.

Methode 3: Bestimmung der antimikrobiellen Aktivität in Abhängigkeit von der Thermobiotikakonzentration

Hierbei wurde in Glas- oder Plastikbehälter eine Mischung aus gleichen Volumina Thermobiotikalösung und Sporensuspension ($10^5$ Sporen je Ansatz) zugegeben. Die Thermobiotikalösungen waren unterschiedlich stark verdünnt. Man trocknete den Behälterinhalt 24 Stunden bei 37°C. Die Biotests wurden bei 120°C ca. 15 Minuten autoklaviert.
Nach Zugabe gleicher Mengen des oben beschriebenen Nährmediums wurden die Ansätze unter geeigneten Bedingungen kultiviert. Ein Farbumschlag des Indikators zeigte das Vorliegen von Sporen an.

Beispiel 1: Herstellung eines antimikrobiellen Mittels aus einer Kultur eines thermophilen Bakteriums

Eine Kultur des thermophilen Bakteriums B. stearothermophilus, Stamm BKM B-718, wurde auf festem Fleischpepton-Agarmedium (1 Gew.-% Fleischpepton, 1,5 Gew.-% Agar, 0,5 Gew.-% NaCl, pH 7,2 ± 0,1, 20 min bei 121°C autoklaviert; Agarschalen mit 9cm Durchmesser) ausgesät. Die angeimpften Agarkulturen wurden in einem Kulturschrank bei 55°C 18 Stunden gehalten. Der mikrobielle Ertrag wurde mit sterilem, gekühltem (+4°C) destillierten Wasser (3 ml pro Agar-Schale) vom festen Nährmedium in ein Zentrifugenglas gespült. Die dabei anfallende Mikrobensuspension wurde 30 Minuten bei 3000 Upm zentrifugiert. Der Überstand wurde dann 5 Minuten bei 120°C autoklaviert. Der Feststoffgehalt des Präparats nach Trockung bei 37°C betrug 12 mg/ml.
Zur Aktivitätsbestimmung pipettierte man in Eppendorf-Gläser jeweils 0,02 ml eines Gemisches aus gleichen Mengen der oben erhaltenen Thermobiotikalösung in unterschiedlicher Verdünnung und einer Sporensuspension von B. stearothermophilus ($10^6$ Sporen). Von jeder Verdünnung wurden 10 identische Ansätze vorbereitet. Die vorbereiteten Testansätze wurden 24 Stunden bei 37°C getrocknet. Die Kontrolltests enthielten anstelle des Thermobiotika-Präparats destilliertes Wasser und wurden ingleicher Weise getrocknet, wie die Testansätze.
Die Biotests wurden 10 Minuten bei 120°C autoklaviert und auf Raumtemperatur abgekühlt. In die Eppendorf-Gläser wurden danach je 0,5 ml des obigen flüssigen Indikator-haltigen Nährmediums zugegeben und die Proben wurden 48 Stunden bei 55°C thermostatisiert. Anhand der Verfärbung des Nährmediums wurden die Tests ausgewertet. Man erhielt folgende Ergebnisse:

| Verdünnung | - | 1:100 | 1:10000 |
|---|---|---|---|
| Aktivitätsindex (%) | 100 | 25 | 0 |

Aus den Prüfergebnissen folgt, daß das Thermobiotika-Präparat von B. stearothermophilus ohne Verdünnung aktiv war, bei 1:100 Vedünnung seine Aktivität sank und bei 1:10000 Verdünnung inaktiv war.
Die signifikant schnellere Beseitigung von Sporen durch Autoklavierung in Gegenwart des erfindungsgemäßen Therobiotikums wird durch folgende Versuchsergebnisse veranschaulicht. Es wurde obige Methode 1 angewendet:

| Sterilisationsdauer (min)[1) | 0 | 5 | 10 | 15 | 20 |
|---|---|---|---|---|---|
| | Anzahl positiver Tests | | | | |
| mit Thermobiotikum | 10[2) | 10 | 0 | 0 | 0 |
| ohne Thermobiotikum[3) | 10 | 10 | 10 | 3 | 1 |

[1) Sterilisation bei 120°C
[2) Inkubation: 48 Stunden, 55°C, 10 Ansätze je Testreihe
[3) Thermobiotikum durch gleiches Volumen steriles Wasser ersetzt

Beispiel 2: Herstellung eines antimikrobiellen Mittels aus einer Kultur eines mesophilen Bakteriums

Eine Kultur des mesophilen Bakteriums Bacillus cereus wurde auf festem Fleischpepton-Agarmedium (1 Gew.-% Fleischpepton, 1,5 Gew.-% Agar, 0,5 Gew.-% NaCl, pH 7,2 ± 0,1, 20 min bei 121°C autoklaviert; Agarschale mit 9 cm Durchmesser) ausgesät. Die angeimpften Agarkulturen wurden in einem Kulturschrank bei 37°C 18 Stunden gehalten. Der mikrobielle Ertrag wurde mit gekühltem destillierten Wasser (3 ml pro Agar-Schale) vollständig in ein Zentrifugenglas gespült. Die dabei anfallende Mikrobensuspension wurde 30 Minuten bei 3000 Upm zentrifugiert. Der Überstand wurde dann 5 Minuten bei 120°C autoklaviert. Der Feststofgehalt nach Trocknung bei 37°C betrug 11mg/ml.

Zur Aktivitätsbestimmung pipettierte man in Eppendorf-Gläser jeweils 0,02 ml eines Gemisches aus gleichen Mengen der oben erhaltenen Thermobiotikalösung in unterschiedlicher Verdünnung und einer Sporensuspension von B. stearothermophilus ($10^6$ Sporen). Für jede Verdünnung verwendete man 10 identische Ansätze. Die vorbereiteten Testansätze wurden 24 Stunden bei 37°C getrocknet. Die Kontrolltests enthielten anstelle des Thermobiotika-Präparats destilliertes Wasser und wurden in gleicher Weise getrocknet wie die Testansätze.

Die Biotests wurden 10 Minuten bei 120°C autoklaviert und auf Raumtemperatur abgekühlt. In die Eppendorf-Gläser wurden danach je 0,5 ml des obigen Indikator-haltigen Nährmediums zugegeben und die Proben wurden 48 Stunden bei 55°C thermostatisiert. Anhand der Verfärbung des Nährmediums wurden die Tests ausgewertet.

Es zeigte sich, daß das Thermobiotika-Präparat von B. cereus bis zur Verdünnung von 1: 10000 (maximale geprüfte Verdünnung) effektiv war (Aktivitätsindex: 100%). Ohne Temperaturerhöhung auf 55°C waren die Thermobiotika gegenüber den Sporen nicht wirksam. Kontrolltests, bei denen nur Sporen von B. stearothermophilus verwendet wurden, enthielten nach dem Erhitzen auf 55°C lebensfähige Mikroben. In Tests mit Sporen und Thermobiotika entwickelten sich nach der Erhitzung keine lebensfähigen Bakterien.

Beispiel 3: Herstellung eines antimikrobiellen Mittels aus einer Kultur eines psychrophilen Bakteriums

Eine Kultur des psychrophilen Yersinia pseudotuberculosis wurde auf festem Fleischpepton-Agarmedium (1 Gew. -% Fleischpepton, 1,5 Gew.-% Agar, 0,5 Gew.-% NaCl, pH 7,2 ± 0,1, 20 min bei 121°C autoklaviert; Agarschale mit 9 cm Durchmesser) ausgesät. Die angeimpften Agarkulturen wurden in einem Kulturschrank bei 10°C 96 Stunden gehalten. Der mikrobielle Ertrag wurde mit gekühltem destillierten Wasser (2 ml pro Agar-Schale) vollständig in ein Zentrifugenglas gespült. Die dabei anfallende Mikrobensuspension wurde 40 Minuten bei 3000 Upm zentrifugiert. Der Überstand wurde dann 5 Minuten bei 120°C autoklaviert. Der Feststoffgehalt des Präparats betrug nach dem Trocknen bei 37°C 10 mg/ml.

Zur Aktivitätsbestimmung pipettierte man in Probengläschen jeweils 0,1 ml des Thermobiotika-Präparats und 1 ml einer 18-stündigen Kultur von E. coli ($2*10^4$ Mikrobenzellen pro Milliliter) vermischt. Die Gläschen ließ man unterschiedlich lang in einem Wasserbad bei 52°C stehen. Als Kontrollen verwendete man einen Ansatz, bei dem Wasser anstelle des Thermobiotikums zugegeben worden war sowie eine Mischung aus den Thermobiotikum und Bakterien, die ohne Erhitzung getestet wirden. Nach Beendigung einer jeden Exposition wurde ein Aliquot (0,1 ml) jeder Mischung auf einer Agar-Platte ausgesät und 72 Stunden im Thermostaten bei 37°C inkubiert. Anschließend wurde die Koloniezahl bestimmt. Die Ergebnisse sind in folgender Tabelle zusammengefaßt:

| Agens | Exposition der Mischung bei 52°C (in Min.) | | | | |
|---|---|---|---|---|---|
| | 0 | 10 | 20 | 30 | 60 |
| | Durchschittliche Koloniezahl | | | | |
| Wasser | ca. 700 | 465 | 326 | 440 | 238 |
| Thermobiotikum aus Y. pseudotubercu-losis | ca. 700 | 210 | 137 | 125 | 78 |

Aus den Versuchsergebnissen folgt, daß sich die durchschnittliche Anzahl der Kolonien bei Erhitzen in Gegenwart des erfindungsgemäßen Thermobiotikums stärker verringerte, als bei Erhitzen ohne erfindungsgemäßem Präparat.

Außerdem wurden Sporen von B. stearothermophilus auf ihr Ansprechen auf obiges Thermobiotikum getestet. Jeweils $10^6$ Sporen wurden unterschiedlich lange bei 120°C mit dem Thermobiotikum inkubiert (obige Methode 1). Je Temperatur wurden 5 Ansätze getestet. Man erhielt folgende Ergebnisse:

| Sterilisationsdauer (min) | 0 | 5 | 10 | 15 |
|---|---|---|---|---|
| | Anzahl positiver Tests | | | |
| mit Thermobiotikum | 5 | 4 | 0 | 0 |
| ohne Thermobiotikum[1] | 5 | 5 | 4 | 0 |

[1] Thermobiotikum wurde durch gleiches Volumen steriles Wasser ersetzt.

Das Thermobiotikum aus Y. pseudotuberculosis bewirkt eine deutlich schnellere Beseitigung der Sporen.

Beispiel 4: Vergleich der Wirksamkeit des Thermobiotikums aus B. stearothermophilus gegenüber homologen Sporen und homologen vegetativen Zellen

Eine Besonderheit des Thermobiotikums aus vegetativen Zellen von B. stearothermophilus besteht in dessen stärker ausgeprägten Wirkung auf homologe Sporen als auf homologe vegetative Zellen. Dies wird durch die in folgender Tabelle zusammengefaßten Versuchsergebnisse veranschaulicht.

| | Expositionszeit | 24 Stunden | | | 3 Stunden | | | 15 Minuten | | | |
|---|---|---|---|---|---|---|---|---|---|---|---|
| | Temperatur (°C) | 4 | 37 | 55 | 70 | 85 | 100 | 105 | 110 | 115 | 120 |
| Sporen | Positive Kontrolltests P | 31 | 31 | 31 | 29 | 32 | 30 | 32 | 23 | 10 | 3 |
| | Positive Tests Q | 33 | 33 | 33 | 33 | 35 | 32 | 30 | 17 | 1 | 0 |
| | P-Q | -2 | -2 | -2 | -4 | -3 | -2 | 2 | 6 | 9 | 3 |
| | Aktivitätsindex A | -6 | -6 | -6 | -14 | -9 | -7 | 6 | 26 | 90 | 100 |
| Vege-tative Zellen | Positive Kontrolltests P | 17 | 16 | 13 | 21 | 27 | 21 | 18 | 12 | 0 | 0 |
| | Positive Tests Q | 15 | 16 | 16 | 17 | 16 | 11 | 17 | 5 | 0 | 0 |
| | P-Q | 2 | 0 | -3 | 4 | 11 | 10 | 1 | 7 | - | - |
| | Aktivitätsindex A | 12 | 0 | -19 | 19 | 41 | 48 | 6 | 58 | - | - |

EP 0 998 938 B1

EP 0 998 938 B1

Bei diesem Versuch wurden für jede Temperatur $10^6$, $10^5$, $10^4$, $10^3$, $10^2$, $10^1$ und $10^0$ Sporen von B. stearothermophilus in 5 Tests und 5 Kontrolltests verwendet. Vegtative Zellen von B. stearothermophilus wurden je Temperatur in Mengen von $10^7$, $10^6$, $10^5$, $10^4$, $10^3$, $10^2$ und $10^1$ Zellen in 5 Tests und 5 Kontrolltests eingesetzt. Es wurde die Gesamtzahl der positiven Tests Q und Kontrolltests P bestimmt. Bei 3-stündiger Exposition war eine signifikante Inhibition auf die vegetativen Zellen schon bei einer Temperatur von 85°C zu beobachten. Bei 15-minütiger Exposition wurden die Sporen durch die Thermobiotika bei Temperaturen über 110°C inhibiert.

Beispiel 5: Inhibition der Sporen von B. stearothermophilus durch Thermobiotika heterologer vegetativer Zellen

Thermobiotika, die bei Kultivierung von Bacillus subtilis, Stamm 168 und Bacillus licheniformis, Stamm G erhalten wurden, wirkten in Kombination mit erhöhter Temperatur inhibierend auf Sporen von B. stearothermophilus.

| Sterlisationsdauer[1] (min) | 0 | 5 | 10 | 15 | 20 |
|---|---|---|---|---|---|
| | Anzahl positiver Tests | | | | |
| Kontrolle | 5[2] | 5 | 5 | 4 | 1 |
| Thermobiotikum aus B. subtilis | 5 | 5 | 0 | 0 | 0 |
| Thermobiotikum aus B. licheniformis | 5 | 5 | 3 | 2 | 0 |

[1]Sterilisationstemperatur: 120°C
[2]Je Testreihe wurden 5 Ansätze untersucht

Obige Ergebnissen zeigen, daß Sporen auch von Thermobiotika heterologer vegetativer Zellen inhibiert werden können.

Beispiel 6: Thermobiotika aus nicht-sporenbildenen Bakterien

Mikroorganismen, die keine Sporen bilden, sind ebenfalls Quellen für Thermobiotika. So sind z.B. aus Zellen der Pestmikrobe Yersinia pestis, die bei 28°C kultiviert werden, Thermobiotika isolierbar, die Sporen von B. stearothermophilus bei 120°C inhibieren.

| Sterlisationsdauer[1] (min) | 0 | 5 | 10 | 15 |
|---|---|---|---|---|
| | Anzahl positiver Tests | | | |
| Kontrolle | 5[2] | 5 | 4 | 0 |
| Thermobiotikum | 5 | 0 | 0 | 0 |
| Thermobiotikum (1:10) | 5 | 5 | 0 | 0 |

[1]Sterilisationstemperatur: 120°C
[2]je Testreihe 5 Ansätze

Diese Ergebnisse bestätigen, daß auch Zellen nichtsporenbildender Bakterien Quelle für ein sporeninhibierendes Thermobiotikum sein können.

Beispiel 7: Herstellung eines Thermobiotikums aus Escherichia coli und Vergleich dessen Aktivität mit Thermobiotika anderer sporenbildender und nicht-sporenbildender Bakterien

Escherichia coli, Stamm K-12 wurde analog zu Beispiel 2 kultiviert. Durch Waschen der Kultur, Zentrifugation und Erhitzen der dabei erhaltenen Waschphase wurde ein Thermobiotikum hergestellt und auf seine Aktivität gegen homologe vegetative Zellen getestet. Dazu wurden jeweils $10^2$ vegetative Zellen bei 52°C mit dem Thermobiotikum in verschiedenen Verdünnungen 30 Minuten inkubiert. Man erhielt folgende Ergebnisse:

| Verdünnung | unverd. | $1:10^4$ | $1:10^6$ | $1:10^8$ |
|---|---|---|---|---|
| Aktivitätsindex (%) | 100 | 100 | 90 | 60 |

Außerdem wurde das Thermobiotikum aus E. coli mit Thermobiotika aus B. subtilis (Sporenbildner) und Y.

pseudotuberculosis (Nichtsporenbildner) hinsichtlich ihrer Aktivität gegenüber Sporen von B. stearothermophilus und vegetative Zellen von E. coli verglichen. Die Ergebnisse sind in folgender Tabelle zusammengefaßt:

| Thermobiotika-Quelle | Testkultur | Testbedingungen | | Anzahl positive Tests | Aktivitäts-index |
|---|---|---|---|---|---|
| | | T (°C) | t (min)[1] | | |
| B. subtilis | $10^6$ Sporen von B. subtilis | 120 | 5,10,15,20 | 0 | 100 |
| E. coli | " | 120 | " | 13 | -8 |
| Y. pseudotubercul. | " | 120 | " | 4 | 55 |
| Wasser | " | 120 | " | 12 | - |
| E. coli | $10^2$ vegetative Zellen von E. coli | 52 | 5,10,20,30,60 | 5 | 75 |
| B. subtilis | " | 52 | " | 16 | 20 |
| Wasser | " | 52 | " | 20 | - |

[1] Je Expositionsdauer wurden 5 (bei Versuchen mit Sporen) bzw. 4 (bei Versuchen mit vegetativen Zellen) identische Ansätze getestet. Die Gesamtzahl der Ansätze je Versuchsreihe betrug daher jeweils 20.

EP 0 998 938 B1

Beispiel 8: Chemotherapeutische Behandlung von inoperablem Magen- und Colonkarzinom mit 5-Fluorurazil bzw. 5-Fluorurazil kombiniert mit einem Thermobiotikum haltigen Loposomenpräparat.

Je zwei Gruppen von Magen- und Colonkarzinompatienten erhielten eine Promedikation mit 5-Fluorurazil (5-FU) oder mit einem Liposomenpräparat, enthaltend die gleiche Menge 5-FU in Kombination mit einem erfindungsgemäßen Thermobiotikum-Präparat (1:1 Gemisch von Thermobiotika gemäß Beispiel 2 und 3). Die Tumorpatienten erhielten Thermobiotikum in einer Menge von etwa 100 bis 500 µg je Applikation. 5-FU wurde in einer Menge von 75 mg je Applikation verabreicht.

Nach der Promedikation wurden beide Patienten-Gruppen sowie eine experimentelle Kontrollgruppe einer lokalen Hyperthermie-Behandlung unterzogen. Hierzu wurde die Tumorregion mit Hilfe eines Hyperthermiegeräts (Jasmin, Brukker-Onkocare) 1 Stunde und 40 Minuten auf eine Temperatur von etwa 43,5 bis 47 °C erhitzt.

Der Behandlungserfolg wurde radiologisch überprüft. Die Ergebnisse sind in folgender Tabelle zusammengefaßt.

Röntgenologische Ergebnisse der Chemotherapie von Patienten mit inoperablen Magen- bzw Colonkarzinom mit 5-Floururazil (5-FU) gegebenenfalls in Kombination mit einem erfindungsgemäßen Thermobiotikum-Gemisch, hergestellt aus Thermobiotika gemäß Beispiel 2 und 3

| Tumor | Medikament | Anzahl der Patienten | Vollregression (%) | Teilregression (%) | stabiler Zustand (%) | Progression (%) |
|---|---|---|---|---|---|---|
| Magen-karzinom | 5-FU + Thermobiotikum | 37 | 2 (5,4 ± 3,7) | 12 (32,4 ± 8,8) | 17 (45,9 ± 8,2) | 6 (16,2 ± 6,0) |
| Magen-karzinom | 5-FU | 15 | 0 | 2 (13,3 ± 8,8) | 5 (33,3 ± 12,0) | 8 (53,3 ± 12,9) |
| Colon-karzinom | 5-FU + Thermobiotikum | 16 | 0 | 9 (56,2 ± 12,4) | 7 (43,7 ± 12,4) | 0 |
| Colon-karzinom | 5-FU | 10 | 0 | 2 (20,0 ± 8,0) | 3 (30,0 ± 14,5) | 5 (50,0 ± 15,8) |

EP 0 998 938 B1

<u>Beispiel 9</u>: In vivo-Untersuchung der Effektivität von Thermobiotika unter hyperthermischen Bedingungen auf übertragbare Tumoren bei Mäusen.

Der Versuch wurden an 54 Mäusen (Hybride F1 (CBAxC57b1/6), männlich, Gewicht 22-24 g, Alter 3-6 Monate) vorgenommen. Den Mäusen wurden je $10^6$ Zellen von Lewis Lungenkarzinom (Stamm LLC) in den rechten Hüftmuskel transplantiert. Nach 8 Tagen hatten die Tumoren ein Volumen von $340 \pm 50$ mm$^3$ erreicht. Die Versuchstiere wurden in 3 Gruppen zu je 18 Mäusen (eine Kontrollgruppe K und zwei Versuchsgruppen V1 und V2) aufgeteilt. Die Mäuse der Versuchsgruppen wurden durch Verabreichung einer Nembutallösung (70 mg Wirkstoff pro kg Körpermasse) narkotisiert. 5 bis 7 min nach der Injektion wurden die Tiere in ein Spezialfutteral eingelegt, in welchem die untere Körperhälfte über ein thermostatisiertes Wasserbad (Wassertemperatur 42,5°C) erwärmt werden konnte. Die hyperthermische Behandlung dauerte 30 min. Während die erste Versuchsgruppe V1 lediglich einer hyperthermischen Behandlung unterzogen wurde, erhielten die Tiere der Versuchsgruppe V2 30 min vor der hyperthermischen Behandlung eine Thermobiotikainjektion (i.v., je 0,4 g pro Maus, 2-fache Verdünnung der Ausgangslösung, Thermobiotikum aus einem mesophilen Bakterium, hergestellt gemäß Beispiel 2).

Nach der thermobiotischen Behandlung wurde das Tumorvolumen in regelmäßigen Abständen überprüft. Am 21. Tag wurden die Versuchstiere getötet, die Tumoren wurden entnommen und in Buhen-Lungenlösung fixiert. Außerdem wurde die Anzahl der Metastasen auf der Lungenoberfläche bei den Versuchstieren unter dem Mikroskop bestimmt.

Die Versuchsergebnisse sind in folgender Tabelle zusammengestellt.

| Gruppe | Anzahl Mäuse in Gruppen | | Tumorvolumen cm$^3$ | Tumorgewicht g | Mittl. Anzahl von Metastasen in Lungen |
|---|---|---|---|---|---|
| | am Anfang | am Ende | | | |
| K | 18 | 16 | 3,7±0,5 | 3,42±0,44 | 21±4 |
| V1 | 18 | 18 | 3,2±0,5 | 2,96±0,38 | 16±4 |
| V2 | 18 | 18 | 2,1±0,3 | 1,71±0,37 | 9±3 |

Aus den Versuchsdaten ist ersichtlich, daß bereits die alleinige hyperthermische Behandlung (V1) einen relevanten Einfluß auf den primären Tumor oder die Lungenmetastasen besitzt. Überraschenderweise konnte jedoch festgestellt werden, daß eine Injektion von Thermobiotikum 30 min vor der hyperthermischen Behandlung zu einer signifikanten Tumorschädigung führt. Weiterhin ist die mittlere Anzahl von Metastasen in der Lunge deutlich verringert (V2).

**Patentansprüche**

1.  Verfahren zur Herstellung eines antimikrobiell wirksamen Mittels, umfassend die thermische Behandlung einer nach Kultivierung eines thermophilen, mesophilen oder psychrophilen, gram-positiven oder gram-negativen, aeroben oder anaeroben gegebenenfalls sporenbildenden Bakteriums und Waschen der Bakterienkultur erhältlichen Waschphase.

2.  Verfahren nach Anspruch 1, unfassend die

    a) Kultivierung des Bakteriums;
    b) Abtrennen der Bakterienkultur vom Kulturmedium und Waschen der Bakterienkultur mit einer wässrigen Waschflüssigkeit;
    c) Abtrennung der so erhaltenen Waschphase von den Bakterien; und
    d) thermische Behandlung der Waschphase bei einer Temperatur von wenigstens etwa 120°C.

3.  Verfahren nach Anspruch 2, wobei man weiterhin

    e) die thermisch behandelte Waschphase einer Größenfraktionierung unterzieht und eine Fraktion gewinnt, deren Bestandteile eine Größe im Bereich von weniger als etwa 500 Dalton besitzen.

4.  Verfahren nach Anspruch 2 oder 3, wobei man

f) die thermisch behandelte und gegebenenefalls größenfraktionierte Waschphase zur Trockne einengt.

5. Verfahren nach einem der vorherigen Ansprüche, wobei man die Bakterien von der Waschphase dadurch abtrennt, daß man bei etwa 2000g bis 30000 g zentrifugiert.

6. Verfahren nach einem der vorherigen Ansprüche, wobei man die Bakterienkultur am Ende der logarithmischen oder zu Beginn der stationären Wachstumsphase wäscht.

7. Verfahren nach einem der vorherigen Ansprüche, wobei man als Waschflüssigkeit keimfreies Wasser oder physiologische Kochsalzlösung verwendet.

8. Verfahren nach einem der vorherigen Ansprüche, wobei man ein Bakterium, ausgewählt unter

   a) sporenbildenden Bakterien der Gruppe Bacillus stearothermophilus, Bacillus subtilis, Bacillus licheniformis und Bacillus cereus; oder
   b) nichtsporenbildenen Bakterien der Gruppe Escherichia coli, Yersinia pseudotuberculosis, Yersinia pestis, Mycobacterium tuberculosis, Staphylococcus aureus, Enterococcus faecalis

   kultiviert.

9. Antimikrobiell wirksames Mittel, **dadurch gekennzeichnet, daß** es wenigstens eine antimikrobielle Aktivität besitzt, die ausgewählt ist unter:

   a) einer inhibierenden Wirkung auf Bakteriensporen; und
   b) einer inhibierenden Wirkung auf vegetative bakterielle und/oder vegetativ nichtbakterielle Zellen

   wobei die antimikrobielle Aktivität thermisch kontrollierbar ist.

10. Antimikrobiell wirksames Mittel nach Anspruch 9, **dadurch gekennzeichnet, daß** es vegetative Zellen in einem Temperaturbereich inhibiert, dessen untere Grenztemperatur $T_u$ etwa der höchsten Temperatur entspricht, bei welcher diese Zellen noch vermehrungsfähig sind, und dessen obere Grenztemperatur $T_o$ etwa der höchsten Temperatur entspricht, bei der diese Zellen noch lebensfähig sind.

11. Antimikrobiell wirksames Mittel nach Anspruch 10, **dadurch gekennzeichnet, daß** es
    vegetative Zellen psychrophiler Bakterien in einem Bereich von $T_u = 20°C$ bis $T_o = 45°C$ inhibiert; und/oder
    vegetative Zellen mesophiler Bakterien in einem Bereich von $T_u = 40°C$ bis $T_o = 70°C$ inhibiert; und/oder
    vegetative Zellen thermophiler Bakterien in einem Bereich von $T_u = 70°C$ bis $T_o = 120°C$ inhibiert; und/oder
    Tumorzellen in einem Bereich von $T_u = 41°C$ bis $T_o = 54°C$ inhibiert.

12. Antimikrobiell wirksames Mittel nach Anspruch 10, **dadurch gekennzeichnet, daß** es Sporen bei einer Temperatur im Bereich von etwa 110 bis 130 °C inhibiert.

13. Antimikrobiell wirksames Mittel nach einem der Ansprüche 9 bis 12, **dadurch gekennzeichnet, daß** deren aktive (r) Bestandteil(e) ein Molekulargewicht im Bereich von etwa 60 bis 450 Dalton besitzt (besitzen).

14. Antimikrobiell wirksames Mittel nach einem der Ansprüche 9 bis 13, **dadurch gekennzeichnet, daß** es Bakteriensporen und vegetative Bakterienzellen über einem pH-Bereich von etwa 2 bis 9 inhibiert.

15. Antimikrobiell wirksames Mittel nach einem der Ansprüche 9 bis 14, **dadurch gekennzeichnet, daß** es zumindest Sporen von Bacillus stearothermophilus und/oder vegetative Zellen von Bacillus stearothermophilus und/oder vegetative Zellen von Escherichia coli inhibiert.

16. Antimikrobiell wirksames Mittel nach einem der Ansprüche 9 bis 15, erhältlich nach einem Verfahren gemäß einem der Ansprüche 1 bis 8.

17. Antimikrobiell wirksames Mittel nach einem der Ansprüche 9 bis 16, kombiniert mit wenigstens einer oberflächenaktiven Substanz.

**18.** Pharmazeutische Zusammensetzung, enthaltend in einem pharmazeutisch akzeptablen, festen oder flüssigen Träger eine antimikrobiell wirksame Menge wenigstens eines Mittels nach einem der Ansprüche 9 bis 17.

**19.** Desinfektionslösung, enthaltend in einem üblichen zur Desinfektion geeigneten flüssigen inerten Träger eine antimikrobiell wirksame Menge wenigstens eines Mittels nach einem der Ansprüche 9 bis 17.

**20.** Verwendung eines Mittels nach einem der Ansprüche 9 bis 17 zur Herstellung einer pharmazeutischen Zusammensetzung zur Behandlung oder Prävention lokaler oder systemischer bakterieller Infektionen.

**21.** Verwendung nach Anspruch 20 zur Herstellung einer pharmazeutischen Zusammensetzung zur Behandlung von Verbrennungen, Rotlauf, Hautentzündungen, urogenitalen Infekten, chronischen Infekten innerer Organe, Infektionen des Nasen-Rachenraums, der Augenhöhlen oder des Innenohrs, und zur Behandlung von Sekundärinfektionen.

**22.** Verwendung eines Mittels nach einem der Ansprüche 9 bis 17 zur Herstellung einer pharmazeutischen Zusammensetzung zur Behandlung von Tumoren.

**23.** Verwendung eines Mittels nach einem der Ansprüche 9 bis 17 zur Herstellung einer pharmazeutischen Zusammensetzung, wobei die Verwendung in Kombination mit wenigstens einer weiteren Therapieform ausgewählt unter Chemotherapie, Strahlentherapie und Hyperthermie, erfolgt.

**24.** Verwendung eines Mittels nach einem der Ansprüche 9 bis 17 zur Herstellung einer Zusammensetzung zur Desinfektion oder Sterilisation von Nahrungsmitteln oder medizinischen Geräten oder zur Konservierung von Nahrungsmitteln.

**Claims**

**1.** Process for preparing an agent with an antimicrobial activity, comprising thermally treating a wash phase which is obtainable by cultivation of a thermophilic, mesophilic or psychrophilic, gram-positive or gram-negative, aerobic or anaerobic, optionally spore-forming bacterium and washing the bacterial culture.

**2.** Process according to claim 1, comprising

    a) cultivating the bacterium;
    b) separating the bacterial culture from the culture medium and washing the bacterial culture with an aqueous washing liquid;
    c) separating the wash phase thus obtained from the bacteria; and
    d) thermally treating the wash phase at a temperature of at least about 120°C.

**3.** Process according to claim 2, wherein furthermore

    e) the thermally treated wash phase is subjected to size fractionation and a fraction is recovered the constituents of which have a size in the region of less than about 500 Daltons.

**4.** Process according to claims 2 or 3, wherein

    f) the thermally treated and optionally size fractionated wash phase is evaporated to dryness.

**5.** Process according to one of the preceding claims, wherein the bacteria are separated from the wash phase by centrifuging at about 2000 to 30000 g.

**6.** Process according to one of the preceding claims, wherein the bacterial culture is washed at the end of the logarithmic growth phase or at the start of the stationary growth phase.

**7.** Process according to one of the preceding claims, wherein germ-free water or physiological saline solution is used as the washing liquid.

8. Process according to one of the preceding claims, wherein a bacterium selected from among

   a) spore-forming bacteria of the group *Bacillus stearothermophilus, Bacillus subtilis, Bacillus licheniformis* and *Bacillus cereus;* or
   b) non-spore forming bacteria of the group *Escherichia coli, Yersinia pseudotuberculosis, Yersinia pestis, Mycobacterium tuberculosis, Staphylococcus aureus, Enterococcus faecalis*

   is cultivated.

9. Antimicrobially active agent, **characterised in that** it has at least one antimicrobial activity which is selected from among:

   a) an inhibiting effect on bacterial spores; and
   b) an inhibiting effect on vegetative bacterial and/or vegetative non-bacterial cells,

   the antimicrobial activity being thermally controllable.

10. Antimicrobially active agent according to claim 9, **characterised in that** it inhibits vegetative cells in a temperature range the lower temperature limit $T_u$ of which corresponds substantially to the highest temperature at which these cells are still capable of replication, and the upper temperature limit $T_o$ of which corresponds substantially to the highest temperature at which these cells are still viable.

11. Antimicrobially active agent according to claim 10, **characterised in that** it inhibits vegetative cells of psychrophilic bacteria in a range from $T_u = 20°C$ to $T_o = 45°C$; and/or
    it inhibits vegetative cells of mesophilic bacteria in a range from $T_u = 40°C$ to $T_o = 70°C$; and/or
    it inhibits vegetative cells of thermophilic bacteria in a range from $T_u = 70°C$ to $T_o = 120°C$; and/or
    it inhibits tumour cells in a range from $T_u = 41°C$ to $T_o = 54°C$.

12. Antimicrobially active agent according to claim 10, **characterised in that** it inhibits spores at a temperature in the range from about 110 to 130°C.

13. Antimicrobially active agent according to one of claims 9 to 12, **characterised in that** the active ingredient(s) thereof has (have) a molecular weight in the range from about 60 to 450 Daltons.

14. Antimicrobially active agent according to one of claims 9 to 13, **characterised in that** it inhibits bacterial spores and vegetative bacterial cells over a pH range from about 2 to 9.

15. Antimicrobially active agent according to one of claims 9 to 14, **characterised in that** it inhibits at least spores from *Bacillus stearothermophilus* and/or vegetative cells of *Bacillus stearothermophilus* and/or vegetative cells of *Escherichia coli.*

16. Antimicrobially active agent according to one of claims 9 to 15 which may be obtained by a process according to one of claims 1 to 8.

17. Antimicrobially active agent according to one of claims 9 to 16, combined with at least one surfactant.

18. Pharmaceutical composition containing, in a pharmaceutically acceptable solid or liquid carrier, an antimicrobially active amount of at least one agent according to one of claims 9 to 17.

19. Disinfectant solution containing in a conventional inert liquid carrier suitable for disinfection an antimicrobially effective amount of at least one agent according to one of claims 9 to 17.

20. Use of an agent according to one of claims 9 to 17 for preparing a pharmaceutical composition for the treatment or prevention of local or systemic bacterial infections.

21. Use according to claim 20 for preparing a pharmaceutical composition for the treatment of bums, erysipelas, skin inflammation, urogenital infections, chronic infections of internal organs, infections of the naso-pharyngeal cavity, the eye sockets or the inner ear and for the treatment of secondary infections.

**22.** Use of an agent according to one of claims 9 to 17 for preparing a pharmaceutical composition for treating tumours.

**23.** Use of an agent according to one of claims 9 to 17 for preparing a pharmaceutical composition, said use being in conjunction with at least one other form of therapy selected from among chemotherapy, radiation therapy and hyperthermia.

**24.** Use of an agent according to one of claims 9 to 17 for preparing a composition for disinfecting or sterilising foodstuffs or medical equipment or for preserving foodstuffs.

**Revendications**

**1.** Procédé de préparation d'un agent efficace du point de vue antimicrobien, comprenant le traitement thermique d'une phase de lavage pouvant être obtenue après culture d'une bactérie thermophile, mésophile ou psychrophile, Gram-positive ou Gram-négative, aérobie ou anaérobie, formant éventuellement des spores, et lavage de la culture bactérienne.

**2.** Procédé selon la revendication 1, comprenant

a) la culture de la bactérie ;
b) la séparation de la culture bactérienne du milieu de culture et le lavage de la culture bactérienne avec un liquide de lavage aqueux ;
c) la séparation de la phase de lavage ainsi obtenue d'avec les bactéries ; et
d) le traitement thermique de la phase de lavage à une température d'au moins environ 120°C.

**3.** Procédé selon la revendication 2, où

e) on soumet en outre la phase de lavage traitée thermiquement à un fractionnement selon la taille et on obtient une fraction dont les constituants possèdent une taille dans le domaine de moins d'environ 500 daltons.

**4.** Procédé selon la revendication 2 ou 3, où

f) on concentre à siccité la phase de lavage traitée thermiquement et éventuellement fractionnée selon la taille.

**5.** Procédé selon l'une des revendications précédentes, où on sépare les bactéries de la phase de lavage en centrifugeant à environ 2 000 g à 30 000 g.

**6.** Procédé selon l'une des revendications précédentes, où on lave la culture bactérienne à la fin de la phase de croissance logarithmique ou au début de la phase de croissance stationnaire.

**7.** Procédé selon l'une des revendications précédentes, où on utilise comme liquide de lavage de l'eau sans germes ou du sérum physiologique.

**8.** Procédé selon l'une des revendications précédentes, où on cultive une bactérie choisie parmi

a) les bactéries formant des spores du groupe de Bacillus stearothermophilus, Bacillus subtilis, Bacillus licheniformis et Bacillus cereus ; ou
b) les bactéries ne formant pas de spores du groupe de Escherichia coli, Yersinia pseudotuberculosis, Yersinia pestis, Mycobacterium tuberculosis, Staphylococcus aureus, Enterococcus faecalis.

**9.** Agent efficace du point de vue microbien, **caractérisé en ce qu'**il possède au moins une activité antimicrobienne qui est choisie parmi :

a) une action inhibitrice sur les spores bactériennes ; et
b) une action inhibitrice sur les cellules bactériennes végétatives et/ou les cellules non bactériennes végétatives

où l'activité antimicrobienne est contrôlable thermiquement.

**10.** Agent efficace du point de vue microbien selon la revendication 9, **caractérisé en ce qu'**il inhibe les cellules végétatives dans un domaine de température dont la température limite inférieure $T_u$ correspond sensiblement à la plus haute température à laquelle ces cellules sont encore capables de se multiplier et dont la température limite supérieure $T_o$ correspond sensiblement à la plus haute température à laquelle ces cellules sont encore capables de vivre.

**11.** Agent efficace du point de vue antimicrobien selon la revendication 10, **caractérisé en ce qu'**il inhibe les cellules végétatives de bactéries psychrophiles dans un domaine de $T_u = 20°C$ à $T_o = 45°C$ ; et/ou

inhibe les cellules végétatives de bactéries mésophiles dans un domaine de $T_u = 40°C$ à $T_o = 70°C$ ; et/ou

inhibe les cellules végétatives de bactéries thermophiles dans un domaine de $T_u = 70°C$ à $T_o = 120°C$ ; et/ou

inhibe les cellules tumorales dans un domaine de $T_u = 41°C$ à $T_o = 54°C$.

**12.** Agent efficace du point de vue antimicrobien selon la revendication 10, **caractérisé en ce qu'**il inhibe les spores à une température dans le domaine d'environ 110 à 130°C.

**13.** Agent efficace du point de vue antimicrobien selon l'une des revendications 9 à 12, **caractérisé en ce que** son ou ses constituants actifs possèdent une masse moléculaire dans le domaine d'environ 60 à 450 daltons.

**14.** Agent efficace du point de vue antimicrobien selon l'une des revendications 9 à 13, **caractérisé en ce qu'**il inhibe les spores bactériennes et les cellules bactériennes végétatives dans un domaine de pH d'environ 2 à 9.

**15.** Agent efficace du point de vue antimicrobien selon l'une des revendications 9 à 14, **caractérisé en ce qu'**il inhibe au moins les spores de Bacillus stearothermophilus et/ou les cellules végétatives de Bacillus stearothermophilus et/ou les cellules végétatives de Escherichia coli.

**16.** Agent efficace du point de vue antimicrobien selon l'une des revendications 9 à 15 pouvant être obtenu par un procédé selon l'une des revendications 1 à 8.

**17.** Agent efficace du point de vue antimicrobien selon l'une des revendications 9 à 16 combiné avec au moins une substance tensioactive.

**18.** Composition pharmaceutique contenant dans un support solide ou liquide pharmaceutiquement acceptable une quantité efficace du point de vue antimicrobien d'au moins un agent selon l'une des revendications 9 à 17.

**19.** Solution désinfectante contenant dans un support inerte liquide approprié à la désinfection courant une quantité efficace du point de vue antimicrobien d'au moins un agent selon l'une des revendications 9 à 17.

**20.** Utilisation d'un agent selon l'une des revendications 9 à 17 pour la préparation d'une composition pharmaceutique pour le traitement ou la prévention d'infections bactériennes locales ou systémiques.

**21.** Utilisation selon la revendication 20 pour la préparation d'une composition pharmaceutique pour le traitement des brûlures, du rouget, des inflammations cutanées, des infections urogénitales, des infections chroniques d'organes internes, des infections de la région rhinopharyngée, des orbites ou de l'oreille interne, et pour le traitement des infections secondaires.

**22.** Utilisation d'un agent selon l'une des revendications 9 à 17 pour la préparation d'une composition pharmaceutique pour le traitement des tumeurs.

**23.** Utilisation d'un agent selon l'une des revendications 9 à 17 pour la préparation d'une composition pharmaceutique, où l'utilisation a lieu en combinaison avec au moins une autre forme de thérapie choisie parmi la chimiothérapie, la radiothérapie et l'hyperthermie.

**24.** Utilisation d'un agent selon l'une des revendications 9 à 17 pour la préparation d'une composition pour la désinfection ou la stérilisation d'aliments ou d'appareils médicaux ou pour la conservation d'aliments.